# EUROPEAN PATENT APPLICATION

(11) **EP 4 710 846 A1**
(43) Date of publication of application: **18.03.2026**
(21) Application number: 25201595.3
(22) Date of filing: 11.09.2025
(51) Int. Cl.: A61B 5/0215, A61B 5/00, A61B 17/12

(54) **VASCULAR OCCLUSION CATHETER SYSTEM FOR PARTIAL OCCLUSION OR FULL OCCLUSION**

(30) Priority: 16.09.2024 US 202463694993 P
(71) Applicant: Prytime Medical Devices, Inc., Boerne, TX 78006 (US)
(72) Inventor: Franklin, Curtis J., Boerne, 78006 (US); Krummenacher, Todd J., Boerne, 78006 (US); Spalding, Marshall C., Boerne, 78006 (US); Schmid, Gregory S., Boerne, 78006 (US)
(74) Representative: Westphal, Mussgnug & Partner Patentanwälte mbB

(57) **Abstract**

A vascular occlusion catheter system for at least partial occlusion of a target vessel includes proximal and distal catheter tubular members and an intervening occlusion balloon. A display hub with a display screen is positioned upon the proximal catheter tubular member. A proximal pressure sensor is in fluid communication with an external environment proximal of the balloon. A distal pressure sensor is in fluid communication with an external environment distal of the balloon. The display hub is preset with a respective above and below balloon target blood pressure ranges each associated with safe partial occlusion of the target vessel; configured to output live proximal and distal pressure sensor readings on the display screen; configured to display visual cues on the display screen indicating whether the live proximal and/or distal pressure sensor readings are within the corresponding below and/or above balloon target blood pressure ranges.

## Description

### CROSS-REFERENCE TO RELATED APPLICATION

This application claims priority from U.S. Provisional Patent Application No. 63/694,993, filed September 16, 2024, titled "Resuscitative Endovascular Balloon Occlusion of the Aorta ("REBOA")", the entire contents of which are incorporated by reference herein.

### STATEMENT REGARDING FEDERALLY SPONSORED RESEARCH OR DEVELOPMENT

This invention was made with government support under Contract No. W81XWH2010524 awarded by the Department of Defense. The government has certain rights in the invention.

### BACKGROUND OF THE DISCLOSURE

This disclosure relates to vascular occlusion catheters and, more particularly, to vascular occlusion catheters capable of performing both partial and full/complete vascular occlusion.

Vascular occlusion may be indicated in either the venous system and/or the arterial system. Endo-arterial occlusion is a procedure in which an artery is temporarily at least partially occluded in order to restrict blood flow upstream or downstream of the occlusion site for purposes of a vascular procedure or repair. Partial occlusion of the aorta is beneficial to mitigate the risk of ischemia below (downstream of) the occlusion site to limit the lack of blood flow to organs and tissue below the occlusion site. That is, partial perfusion past the occlusion balloon can provide the benefits of focusing or directing a majority of blood flow to the brain, heart and lungs or other upstream portions of the patient, while also potentially increasing the amount of time the occlusion balloon can be implanted in the patient by providing at least partial blood flow to the patient's organs downstream of the occlusion member, such as to the patient's liver, digestive tract, kidneys and legs.

Conventionally, vascular occlusion procedures are conducted in a medical facility using fluoroscopy or other imaging or monitoring equipment to assist the practitioner in successful execution of the procedure. Vascular occlusion procedures are sometimes required in trauma or emergency situations, however, where a patient is undergoing severe central torso hemorrhaging. Particularly when not in a medical facility, such as on a battlefield or on a public street or highway, imaging and monitoring capability is not available to emergency responders or field medical practitioners. In such high-stress and time sensitive situations, a practitioner may benefit immensely from assistive or guidance measures. Additionally, in a trauma or emergency situations, the operator of the vascular occlusion catheter system may be an untrained individual rather than a vascular occlusion procedure expert. Yet further, not all grades of occlusion are the same. Generally, the more flow past the occlusion balloon, the better the distal perfusion, but also the increased amount of hemorrhage.

One approach to addressing the challenges associated with emergency vascular occlusion procedures is to utilize a vascular occlusion catheter system employing a processor and a closed feedback loop control system configured to automate balloon inflation to the desired degree of vessel occlusion and subsequent maintenance of the desired degree of vessel occlusion. One drawback to such an approach, however, is that any software malfunction, e.g., any software hang or delayed response, could be catastrophic, e.g., fatal. Another drawback to a closed feedback loop control system is the complexity of setup, which involves more parts and connections and steps which may be improperly performed.

It would, therefore, be desirable to design, develop and implement an occlusion balloon catheter system configured with integrated/on-board measures intended to assist or guide a practitioner or novice operator manually operating the catheter system with expeditious and successful execution of a vascular occlusion procedure, and also configured to provide the practitioner or novice operator with increased data to aid with execution of the vascular occlusion procedure in a manner providing increased benefits and reduced ill effects. Such an open loop system, i.e., system provided notifications, but operator performed task(s), provides additional measures of safety for the subject against undesirable or inadvertent physiological states, such as, for example, complete vessel occlusion when partial vessel occlusion is preferred, or, alternatively, insufficient occlusion.

### BRIEF SUMMARY OF THE DISCLOSURE

Briefly stated, one aspect of the present disclosure is directed to a vascular occlusion catheter employed for at least partial occlusion of a target vessel. The vascular occlusion catheter system includes a proximal catheter tubular member; a distal catheter tubular member; and an intervening occlusion balloon connected to the proximal catheter tubular member and the distal catheter tubular member. One of a stiffener member or a hypotube extends within the proximal catheter tubular member, the occlusion balloon and into the distal catheter tubular member, the stiffener member or the hypotube being permanently anchored within the vascular occlusion catheter system. A display hub with a display screen is fixedly positioned upon the proximal catheter tubular member. A proximal pressure sensor, electrically connected with the display hub, is positioned within the proximal catheter tubular member and in fluid communication with an environment external to the vascular occlusion catheter system proximal of the occlusion balloon. A distal pressure sensor, electrically connected with the display hub, is positioned within the distal catheter tubular member and in fluid communication with an environment external to the vascular occlusion catheter system distal of the occlusion balloon. The display hub is: (i) preset with an above balloon target blood pressure range and a below balloon target blood pressure range each associated with safe partial occlusion of the target vessel, (ii) configured to output a live proximal pressure sensor reading and a live distal pressure sensor reading on the display screen, (iii) configured to display a visual cue on the display screen indicating whether the live distal pressure sensor reading is within the above balloon target blood pressure range, and (iv) configured to display a visual cue on the display screen indicating whether the live proximal pressure sensor reading is within the below balloon target blood pressure range.

In one configuration, the live distal pressure sensor reading output includes a first numerical output and the live proximal pressure sensor reading output includes a second numerical output, the visual cue indicating whether the live distal pressure sensor reading is within the above balloon target blood pressure range includes a colored border around the first numerical output and the visual cue indicating whether the live proximal pressure sensor reading is within the below balloon target blood pressure range includes a colored border around the second numerical output.

In any one of the previous configurations, the live distal pressure sensor reading output includes a first numerical output and the live proximal pressure sensor reading output includes a second numerical output, the visual cue indicating whether the live distal pressure sensor reading is within the above balloon target blood pressure range includes a shaded color-coded shape surrounding the first numerical output and the visual cue indicating whether the live proximal pressure sensor reading is within the below balloon target blood pressure range includes a shaded color-coded shape surrounding the second numerical output.

In any one of the previous configurations, the live distal pressure sensor reading output includes a first numerical output and the live proximal pressure sensor reading output includes a second numerical output, the visual cue indicating whether the live distal pressure sensor reading is within the above balloon target blood pressure range includes a color-coded font color of the first numerical output and the visual cue indicating whether the live proximal pressure sensor reading is within the below balloon target blood pressure range includes a color-coded font color of the second numerical output.

In any one of the previous configurations, the live distal pressure sensor reading output includes a first waveform and the live proximal pressure sensor reading output includes a second waveform, the visual cue indicating whether the live distal pressure sensor reading is within the above balloon target blood pressure range includes a color-coded output of the first waveform and the visual cue indicating whether the live proximal pressure sensor reading is within the below balloon target blood pressure range includes a color-coded output of the second waveform.

In any one of the previous configurations, the live distal pressure sensor reading output includes a first waveform and the live proximal pressure sensor reading output includes a second waveform, and the first waveform and the second waveform are output on a single graph.

In any one of the previous configurations, at least one of the above balloon target blood pressure range and the below balloon target blood pressure range is output on the display screen.

In any one of the previous configurations, the display hub includes a segmented timer, the display hub being configured to record occlusions times and divide, categorize and chronologically track duration of time in a particular occlusion state, or a particular grade of an occlusion state, according to data received from the segmented timer, the proximal pressure sensor and the distal pressure sensor. In one configuration, the display hub is configured to classify proximal and distal pressure sensor readings as a no vessel occlusion, occlusion state when the absolute value difference between distal and proximal blood pressure sensor readings is less than approximately 10 mmHg. In either one of the previous configurations, the display hub is configured to classify proximal and distal pressure sensor readings as a partial vessel occlusion, occlusion state when (i) the distal pressure sensor reading is between approximately 90 mmHg and approximately 110 mmHg in combination with the proximal pressure sensor reading being at least approximately 20 mmHg, or (ii) the distal pressure sensor reading is at least approximately 90 mmHg in combination with the proximal pressure sensor reading being at least approximately 20 mmHg. In one configuration, the display hub is configured to classify proximal and distal pressure sensor readings as a first grade of partial vessel occlusion when the proximal pressure sensor reading is between approximately 50 mmHg and approximately 75 mmHg; classify proximal and distal pressure sensor readings as a second grade of partial vessel occlusion when the proximal pressure sensor reading is between approximately 35 mmHg and approximately 49 mmHg; and classify proximal and distal pressure sensor readings as a third grade of partial vessel occlusion when the proximal pressure sensor reading is between approximately 21 mmHg and approximately 34 mmHg.

In any one of the previous configurations, the display hub is configured to classify proximal and distal pressure sensor readings as a partial vessel occlusion, occlusion state when at least one of: (i) the absolute value difference between distal and proximal blood pressure sensor readings is at least approximately 10 mmHg; (ii) a systolic proximal pressure sensor reading is at least 5 mmHg greater than a diastolic proximal pressure sensor reading; and (iii) the proximal pressure sensor reading being greater than approximately 20 mmHg. In one configuration, the display hub is configured to classify proximal and distal pressure sensor readings as a first grade of partial vessel occlusion when the proximal pressure sensor reading is between approximately 50 mmHg and approximately 75 mmHg; classify proximal and distal pressure sensor readings as a second grade of partial vessel occlusion when the proximal pressure sensor reading is between approximately 35 mmHg and approximately 49 mmHg; and classify proximal and distal pressure sensor readings as a third grade of partial vessel occlusion when the proximal pressure sensor reading is between approximately 21 mmHg and approximately 34 mmHg.

In any one of the previous configurations, the display hub is configured to classify proximal and distal pressure sensor readings as a complete vessel occlusion, occlusion state when at least one of: (i) a systolic proximal pressure sensor reading is less than approximately 5 mmHg greater than a diastolic proximal pressure sensor reading; and (ii) the proximal pressure sensor reading is approximately 20 mmHg or less.

In any one of the previous configurations, the display hub is configured to output a timer display as a grid logging a duration of time spent in each occlusion state or grade of the occlusion state.

In any one of the previous configurations, the display hub is configured to display an alert notifying whether an artery is detected according to a comparison of at least one of the proximal pressure sensor reading and the distal pressure sensor reading to a preset threshold pressure associated with the target artery, prior to inflation of the occlusion balloon. In one configuration, the display screen includes a command field and the alert is a text alert displayed within the command field.

In any one of the previous configurations, the vascular occlusion catheter system includes a stiffener member defining at least one tapered section in a direction from a proximal side toward a distal side thereof. In any one of the previous configurations, the vascular occlusion catheter system may alternatively include a stiffener member having a substantially uniform diameter throughout an axial extent thereof, and also include a solid distal wire extending within the distal catheter tubular member, a section of the solid distal wire overlapping with a section of the stiffener member, whereby the solid distal wire has a central axis that is radially offset from a central axis of the stiffener member. In any one of the previous configurations, the vascular occlusion catheter system may alternatively include a hypotube, wherein the distal pressure sensor is electrically connected with the display via an internal lumen of the hypotube.

### BRIEF DESCRIPTION OF THE SEVERAL VIEWS OF THE DRAWINGS

The following description of embodiments of the disclosure will be better understood when read in conjunction with the appended drawings. It should be understood, however, that the disclosure is not limited to the precise arrangements and instrumentalities shown. In the drawings:
Fig. 1 is a perspective view of a vascular occlusion catheter system according to an embodiment of the present disclosure;
Fig. 2 is an enlarged, partial cross-sectional view of a proximal section of the vascular occlusion catheter system of Fig. 1, taken along sectional line 2-2;
Fig. 3 is an enlarged, partial cross-sectional view of a proximal section of an alternative configuration of the vascular occlusion catheter system of Fig. 1, taken along sectional line 2-2;
Fig. 4 is an enlarged, partial cross-sectional view of a proximal section of the occlusion catheter of Fig. 1, taken along a sectional line perpendicular to sectional line 2-2;
Fig. 5 is a partial cross-sectional view of the vascular occlusion catheter system of Fig. 1, taken along sectional line 2-2;
Fig. 6 is an enlarged, partial cross-sectional view of a distal section of the vascular occlusion catheter system of Fig. 1, taken along sectional line 2-2;
Fig. 7 is a further enlarged, partial cross-sectional view of the distal section of Fig. 6, taken along sectional line 2-2;
Fig. 8 is an enlarged, partial cross-sectional view of an alternative configuration of the distal section of the vascular occlusion catheter system of Fig. 1, taken along sectional line 2-2;
Fig. 9 is a further enlarged, partial cross-sectional view of the distal section of Fig. 8, taken along sectional line 2-2;
Fig. 10 is a further enlarged, partial cross-sectional view of the proximal section of Fig. 2, taken along sectional line 2-2;
Fig. 11 is another enlarged, partial cross-sectional view of the proximal section of the vascular occlusion catheter system of Fig. 1, taken along sectional line 2-2;
Fig. 12 is a transparent cross-sectional view of the proximal section of the vascular occlusion catheter system of Fig. 1, taken along sectional line 12-12;
Fig. 13A is a top plan view of one display output of a display screen of a display hub of the vascular occlusion catheter system of Fig. 1;
Fig. 13B is a top plan view of another display output of the display screen of the display hub of the vascular occlusion catheter system of Fig. 1;
Fig. 13C is a top plan view of another display output of the display screen of the display hub of the vascular occlusion catheter system of Fig. 1;
Fig. 13D is a top plan view of another display output of the display screen of the display hub of the vascular occlusion catheter system of Fig. 1; and
Fig. 14 is a top plan view of the display hub of the vascular occlusion catheter system of Fig. 1.

### DETAILED DESCRIPTION OF THE DISCLOSURE

Certain terminology is used in the following description for convenience only and is not limiting. The terms "lower," "bottom," "upper", "top", "above" and "below" describe the orientation, or relative orientation, of various elements as they appear in the drawings to which reference is made. The terms "inwardly," "outwardly," "upwardly" and "downwardly" refer to directions toward and away from, respectively, the geometric center of the catheter system, and designated parts thereof, in accordance with the present disclosure. In describing the catheter system, the term "proximal" indicates a portion or component of the catheter system that is oriented away from the body into which the system is, or is intended to be, placed. In describing the catheter system, the term "distal" indicates a portion or component of the catheter system that is oriented toward the body into which the system is, or is intended to be, placed. Unless specifically set forth herein, the terms "a," "an" and "the" are not limited to one element, but instead should be read as meaning "at least one." The terminology includes the terms noted above, derivatives thereof and terms of similar import.

The term "coupled" and variations thereof, as used herein, means the joining of two members directly or indirectly to one another. Such joining may be stationary (e.g., permanent; fixed) or moveable (e.g., removable; releasable). Such joining may be achieved with the two members coupled directly to each other or with the two members coupled to each other using at least one intervening member, unless otherwise indicated. As one example, if the term "coupled" or variations thereof are modified by an additional term (e.g., directly coupled), the aforementioned, generic definition of "coupled" is modified by the plain language meaning of the additional term (e.g., "directly coupled" means the joining of two members without any separate intervening member) Such coupling may be mechanical, electrical, fluidic a combination thereof, or the like, unless otherwise indicated.

The term "or," as used herein, is used in its inclusive sense (and not in its exclusive sense) so that when used to connect a list of elements, the term "or" means one, some, or all of the elements in the list. Conjunctive language such as the phrase "at least one of X, Y, and Z," unless specifically stated otherwise, is understood to convey that an element may be either X; Y; Z; X and Y; X and Z; Y and Z; or X, Y, and Z (i.e., any combination of X, Y, and Z including just one of them). Thus, such conjunctive language is not generally intended to imply that certain embodiments require at least one of X, at least one of Y, and at least one of Z to each be present, unless otherwise indicated.

The ordinal terms "first," "second," etc., as used herein, are used to distinguish one element from another and do not denote any specific order or sequence, nor do they require the presence of any particular number of elements. It will, therefore, be understood that, unless otherwise indicated, the use of ordinal numbers (e.g., "first," "second") in the claims is not intended to limit the scope of the claims to require the presence of both elements associated with those ordinal numbers. For example, the recitation of a "first widget" does not require the presence of a "second widget."

It should also be understood that the terms "about," "approximately," "generally," "substantially" and like terms, used herein when referring to a dimension or characteristic of a component of the disclosure, indicate that the described dimension/characteristic is not a strict boundary or parameter and does not exclude insubstantial or inconsequential modifications or alterations therefrom that are functionally similar, e.g., +/- 10%. At a minimum, such references that include a numerical parameter would include variations that, using mathematical and industrial principles accepted in the art (e.g., rounding, measurement or other systematic errors, manufacturing tolerances, etc.), would not vary the least significant digit.

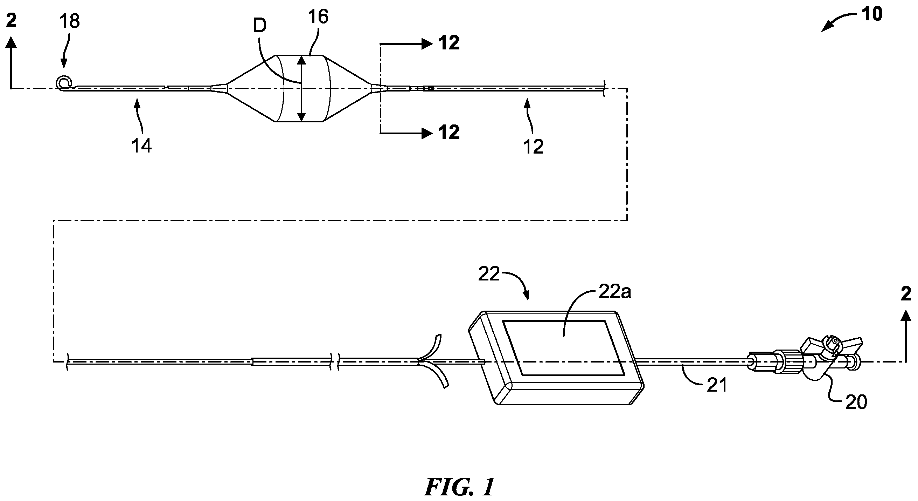

Referring to the drawings in detail, wherein like numerals indicate like elements throughout, there is shown in Figs. 1-14 a vascular occlusion catheter system, generally designated 10, in accordance with an embodiment of the present disclosure. The catheter system 10 includes a proximal catheter tubular member 12, a distal catheter tubular member 14 and an expandable occlusion member, e.g., balloon, 16 mounted to the proximal and distal tubular members 12, 14. In one configuration, the proximal and distal tubular members 12, 14 may take the form of shafts with internal lumens, but the disclosure is not so limited. In one configuration, the catheter system 10 may be inserted into a subject via the common femoral artery, in a retrograde manner relative to blood flow, whereby blood distal to the occlusion balloon 16 is upstream thereof and blood proximal to the occlusion balloon 16 is downstream thereof.

A proximal neck portion 16a of the occlusion balloon 16 may be sealingly mounted/anchored (e.g., via bonding (e.g., adhesive or thermal), welding, a combination thereof or the like) to the proximal catheter tubular member 12, such as described in in U.S. Patent Application Publication No. 2025/0241647 ("the '895 publication"), the entire contents of which are incorporated by reference herein in their entirety. Similarly, a distal neck portion 16b of the occlusion balloon 16 may be sealingly mounted/anchored (e.g., via bonding (e.g., adhesive or thermal), welding, a combination thereof or the like) to the distal catheter tubular member 14. The distal catheter tubular member 14 distally terminates with an atraumatic tip 18. In one configuration, the proximal and distal catheter tubular members 12, 14 may be constructed of medical grade, biocompatible polymers, such as, for example, without limitation, silicone, nylon, polyurethane, PETE, latex, thermoplastic elastomers, polyether block amides (e.g., PEBAX, Arkema, Paris, France), a combination thereof, or the like.

An inflation hub 20 is positioned at a proximal end of the catheter system 10, and operates as a fluid connector to the catheter system 10. That is, a fluid injection and/or withdrawal device (not shown), e.g., a syringe, a pump, a reservoir, a combination thereof, or the like, is selectively connected to the inflation hub 20 to fluidly communicate with the catheter system 10. In one embodiment, the inflation hub 20 includes a flow valve and/or a pressure relief valve (discretely or in combination). As should be understood by those of ordinary skill in the art, a flow valve is two-way valve selectively actuatable between open and closed positions to permit or deny fluid passage therethrough, whereas a pressure relief valve is a one-way valve configured to automatically open at a predetermined threshold pressure to release overpressure to the outside/external environment.

As shown, a display hub 22 is fixedly positioned upon the proximal tubular member 12. The display hub 22 includes a display screen 22a configured to display parameters, status indicators, commands, a combination thereof or the like, as will be described in further detail below. In one non-limiting configuration, the display hub 22 may take the form of a two-halved clamshell enclosure mounted upon the proximal tubular member 12. In one non-limiting configuration, the display hub 22 may include an internal hub frame (not shown). In one non-limiting configuration, the internal hub frame may operate as a structural chassis of the display hub 22, including securing the clamshell enclosure upon the proximal catheter tubular member 12. The internal hub frame may structurally support components of the display hub 22, such as, without limitation, circuit board(s), processor(s), power supply components, other electronics, combinations thereof or the like.

In one non-limiting configuration, the proximal catheter tubular member 12 may originate within the display hub 22 and extend distally therefrom. In such a configuration, the proximal catheter tubular member 12 may be fixedly secured to the internal hub frame. In such a configuration, the inflation hub 20 may be fluidly connected with the proximal catheter tubular member 12 inside the display hub 22, e.g., via an inflation tube 21. Alternatively, the proximal catheter tubular member 12 may originate at the inflation hub 20 and extend through the display hub 22.

The occlusion catheter system 10 also includes an inner catheter shaft, i.e., stiffener member, 24 axially extending within the proximal catheter tubular member 12, the occlusion balloon 16 and terminating within the distal catheter tubular member 14 (as will be described in further detail below). The stiffener member 24 forms the structural backbone/chassis of the catheter system 10. Stated differently, the stiffener member 24, being fixedly integrated into the catheter system 10, forms the primary load-bearing framework of the catheter system 10, providing the catheter system 10 with sufficient structural integrity to withstand the forces applied thereon within the vasculature of a patient during a vascular occlusion procedure, advantageously eliminating the necessity for a standalone, removable guidewire to provide such load bearing stability. To provide the structural backbone, the stiffener member 24 may be constructed of a metal, a metal alloy, a polymer, a combination thereof, e.g., composite braided shafts, or the like. Non-limiting examples of the stiffener member 24 material include stainless steel, nitinol, plastic reinforced with fiberglass, Kevlar^{®} owned by E.I. Du Pont De Nemours and Company, and/or nylon. Generally, the stiffener member 24 forms the most elastic shaft-component of the occlusion catheter system 10, such that the stiffener member 24 substantially returns to its initial configuration after a load is applied thereon and subsequently stopped. Optionally, the stiffener member 24 may be constructed of a super-elastic material (e.g., such as nitinol). Alternatively, however, the stiffener member 24, and at least one of the proximal catheter tubular member 12 and/or the distal catheter tubular member 14 may define substantially the same elasticity.

In one non-limiting configuration, one or both of the proximal catheter tubular member 12 and the stiffener member 24 may be permanently secured/anchored at the respective proximal ends thereof within the display hub 22, e.g., to the internal hub frame, such as, for example, as described in International Patent Application Publication No. WO 2022/197895 ("the '895 publication"), the entire contents of which are incorporated by reference herein in their entirety. As should be understood by those of ordinary skill in the art, the term permanent is intended to cover different attachment mechanisms that are not disconnectable or removable without causing unintended or undesirable damage or disassembly. Examples of permanent attachment include, without limitation, bonding (e.g., adhesive or thermal), welding, jacketing, a combination thereof, or the like. Conversely, in another non-limiting configuration, one or both of the proximal catheter tubular member 12 and the stiffener member 24 may be permanently secured/anchored at the respective proximal ends thereof to the inflation hub 20.

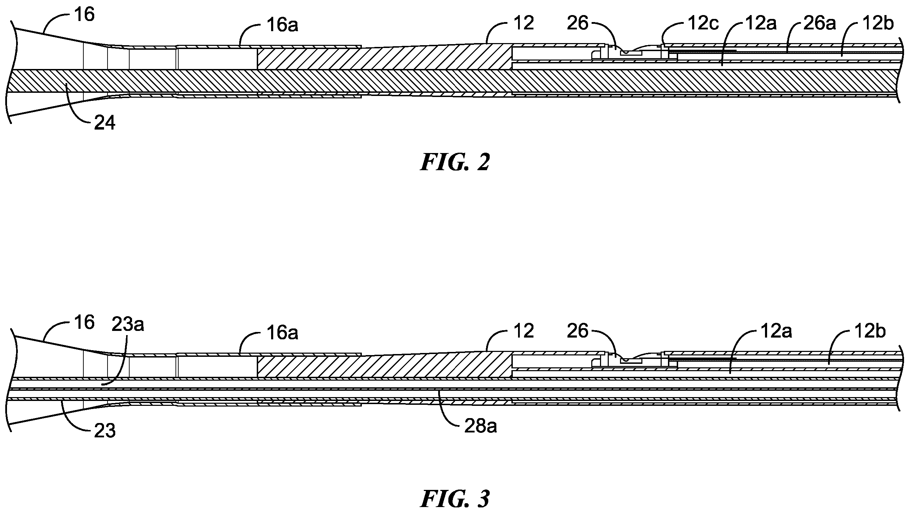

As shown best in Figs. 2-4 and 12, the proximal catheter tubular member 12 may include one or more discrete internal lumens (e.g., multiple, discretely extruded lumens). The stiffener member 24 may extend axially through a first internal lumen 12a of the proximal catheter tubular member 12. The proximal catheter tubular member 12 may also include a second internal lumen 12b, extending generally parallel with the first internal lumen 12a along at least a portion of the extent thereof. In one non-limiting configuration, the second internal lumen 12b distally terminates proximally to the occlusion balloon 16. A proximal sensor 26 may be included in the occlusion catheter system 10, proximal to the occlusion balloon 16, for "below balloon" sensing. In such a configuration, a proximal window 12c may be formed in the sidewall of the proximal catheter tubular member 12, which extends into the second internal lumen 12b. The proximal sensor 26 may be positioned within the proximal window 12c, or otherwise within the second internal lumen 12b, and electrically connected with the display hub 22 via at least one proximal sensor signal wire 26a traveling down the second internal lumen 12b.

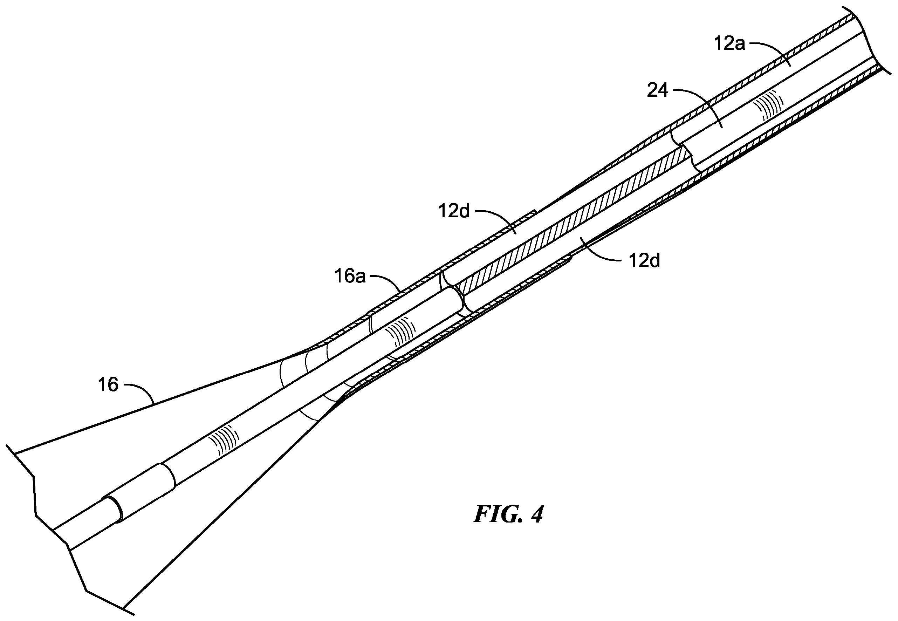

The first internal lumen 12a of the proximal catheter tubular member 12 may operate as an inflation lumen, directly or indirectly in fluid communication with the inflation hub 20 on a proximal side thereof and directly or indirectly in fluid communication with the occlusion balloon 16 on a distal side. As shown best in Fig. 4, the first internal lumen 12a may lead into at least one, internal transition lumen 12d extending parallel to the stiffener member 24. Accordingly, inflation of the occlusion balloon 16 may be provided via the free space of the proximal shaft lumen 12a unoccupied by the stiffener member 24. If employed, the internal transition lumen(s) 12d are fluidly disconnected from the second internal lumen 12b.

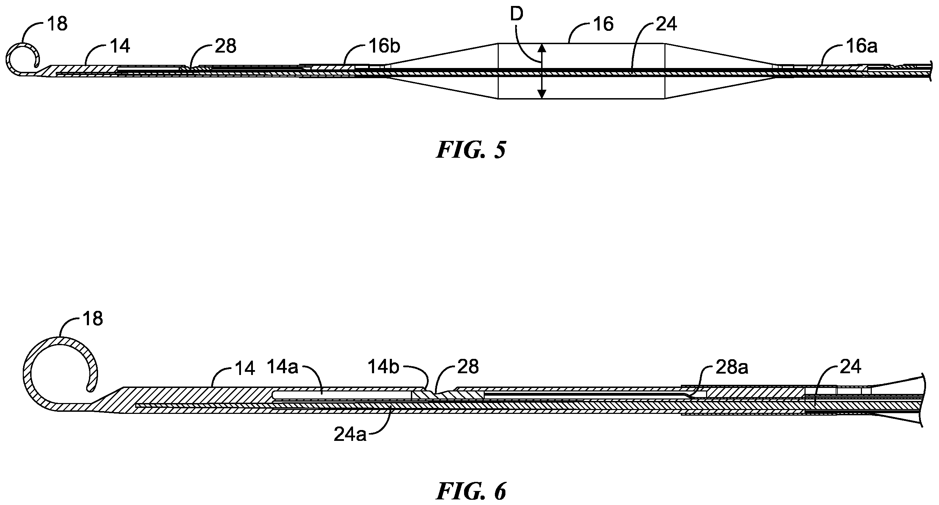

Turning to the distal side of the occlusion balloon 16 (Figs. 6-10), the distal catheter tubular member 14 includes a distal internal lumen 14a extending along at least a portion of the distal catheter tubular member 14. In one non-limiting configuration, a distal sensor 28 may also be employed in the occlusion catheter system 10, distal to the occlusion balloon 16, for "above balloon" sensing. In such a configuration, a distal window 14b may be formed in the sidewall of the distal catheter tubular member 14, which extends into the distal internal lumen 14a. The distal sensor 28 may be positioned within the distal window 14b, or otherwise within the internal distal lumen 14a, and electrically connected with the display hub 22 with a distal sensor signal wire 28a, as will be described in further detail below.

As should be understood by the those of ordinary skill in the like, in configurations where at least one of a proximal sensor 26 and a distal sensor 28 is employed, one or both of the sensors 26, 28 may take the form of a pressure sensor, a flow sensor, a chemical sensor, a temperature sensor, an oxygenation sensor, a heart rate sensor, an ischemia sensor, a biological sensor, a combination thereof, or the like, i.e., a sensor for another physiological parameter. In configurations where both a proximal sensor 26 and a distal sensor 28 are employed, each sensor may be configured to detect the same or different physiological parameter(s).

Turning again to the stiffener member 24, and as shown in Figs. 2-12, the stiffener member 24 is solid, i.e., a solid shaft without an internal lumen extending along the axial extent thereof within the proximal catheter tubular member 12, the occlusion balloon 16 and into the distal catheter tubular member 14. Advantageously, a solid stiffener member 24 is cost effective to manufacture, thereby contributing to cost reduction of catheter system manufacture. Further advantageously, a solid/lumen-less stiffener member 24 may define a reduced cross-sectional profile. Such reduced cross-sectional profile contributes to achieving an ultimate cross-sectional profile of the proximal catheter tubular member 12, the distal catheter tubular member 14 and the occlusion balloon 16 in an uninflated state, of less than 8 Fr, e.g., 7 Fr, 6 Fr, 5 Fr or 4 Fr. As should be understood by those of ordinary skill in the art, the narrower the catheter, the narrower the required introducer sheath, which, in turn, requires a smaller access site incision in the patient's body. Advantageously, the smaller the access site incision, the shorter the amount of time required to close the incision after completion of the vascular occlusion procedure, and fewer prospective complications for the subject.

In one configuration, as shown best in Figs. 5-7, the stiffener member 24 extends distally beyond a distal extent of the distal internal lumen 14a, e.g., proximate to a base end of the atraumatic tip 18. In one such configuration, the stiffener member 24 may define at least one tapered section 24a in a direction from the proximal side toward the distal side thereof. In one such configuration, the stiffener member 24 may define a generally uniform cross-sectional dimension throughout the proximal catheter tubular member 12 and the occlusion balloon 16 and begin to taper within the distal catheter tubular member 14. Alternatively, the taper or series of tapers could initiate anywhere along the stiffener member 24. Advantageously, such tapering provides a progressive reduction in stiffness of the distal catheter tubular member 14 from the proximal end thereof to the distal end thereof, e.g., due to a natural progressive strain relief proportional to the decreasing thickness of the stiffener member 24, equipping the distal end of the catheter system 10 with increased maneuverability and steerability. Additionally, reduced bending stiffness resulting from such tapering reduces the risk of blood vessel wall perforation. Further advantageously, employing a single, solid stiffener member 24 within the catheter system 10 reduces the total number of components within the catheter system 10, thereby requiring fewer component suppliers, less assembly, less bioburden, and less prospective failure points.

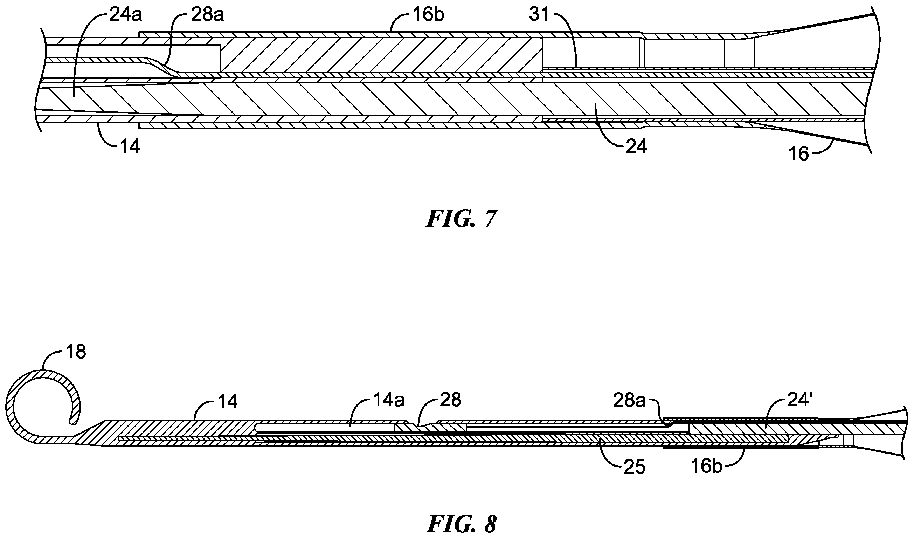

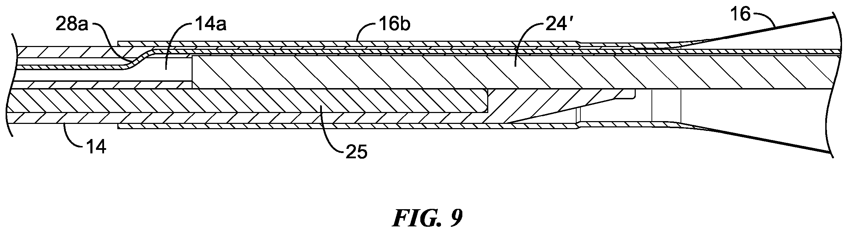

In an alternative configuration, as shown best in Figs. 8 and 9, the stiffener member 24' may define a shorter distal extent than the stiffener member 24. As one example, the stiffener member 24' may terminate within a proximal side of the distal catheter tubular member 14, e.g., within the distal neck portion 16b of the occlusion balloon 16 or within an axial extent of the distal internal lumen 14a. In contrast to the stiffener member 24, the stiffener member 24' may define a substantially uniform diameter throughout the axial extent thereof, i.e., not exhibiting a tapered cross-sectional dimension. A separate solid distal wire 25 may be embedded in the distal catheter tubular member 14, adjacent to, and generally parallel with, the stiffener member 24'. The solid distal wire 25 may be constructed of the same material as the stiffener member 24' or may be constructed of another one of the suitable stiffener member materials previously identified.

A proximal portion of the solid distal wire 25 may overlap with a distal portion of the stiffener member 24'. Stated differently, the solid distal wire 25 may have a central axis that is radially offset from a central axis of the stiffener member 24'. Optionally, at least a section of the proximal portion of the solid distal wire 25 overlapping with the distal portion of the stiffener member 24' may be jacketed to the stiffener member 24' by a polymeric sleeve or by the distal catheter tubular member 14 material itself. The solid distal wire 25 may additionally or alternatively be affixed to the stiffener member 24', e.g., via bonding (e.g., adhesive or thermal), welding, a combination thereof or the like. The solid distal wire 25 may be a tapered wire, extending toward a distal end of the distal catheter tubular member 14, e.g., to a base of the atraumatic tip 18, and tapering in a direction from a proximal end thereof to a distal end thereof, thereby progressively reduce stiffness of the distal catheter tubular member 14 from the proximal end thereof to the distal end thereof as previously described. Alternatively, the solid distal wire 25 may be of substantially uniform diameter throughout the axial extent thereof.

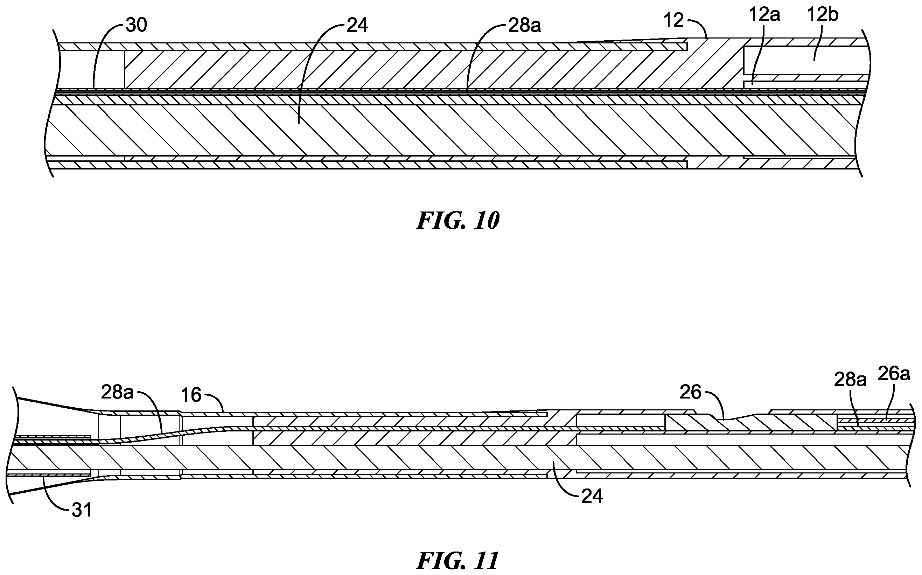

To electrically connect the distal sensor 28 with the display hub 22, the distal sensor signal wire(s) 28a may be routed from the distal sensor 28 (through the distal internal lumen 14a) and along an outer wall of the stiffener member 24, 24' at least through the occlusion balloon 16. Within the proximal catheter tubular member 12, the distal sensor signal wire(s) 28a may travel with the stiffener member 24, 24' through the first internal lumen 12a (see Fig. 10) into electrical communication with the display hub 22. Alternatively, the distal sensor signal wire(s) 28 may divert from the stiffener member 24 and route into the second internal lumen 12b (see Fig. 11) and into electrical communication with the display hub 22. Further alternatively, the distal sensor signal wire(s) 28a may travel down an independent lumen (not shown) within the proximal catheter tubular member 12.

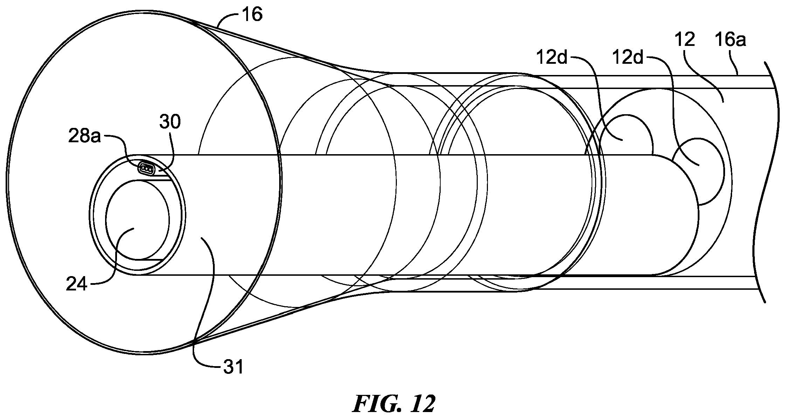

With the distal sensor signal wire(s) 28a traveling along the outside of the stiffener member 24, 24', protection of the distal sensor wire(s) 28a from damage, e.g., resulting from tension on the wire(s) upon catheter bending, may be implemented. As one example, slack may be provided to the wire(s) 28a before fixation at the distal and proximal balloon neck portions 16a, 16b, i.e., fixation between the proximal neck portion 16a, the proximal catheter tubular member 12, and the stiffener member 24, 24' and/or fixation between the distal neck portion 16b, the distal catheter tubular member 12 and the stiffener member 24, 24', e.g., as described in the '895 publication. Optionally, to further protect the sensor wire(s) 28a from damage, the distal sensor signal wire(s) 28a may be run through a protective tube 30 (Fig. 12). In one non-limiting configuration, the protective tube 30 may extend substantially the entire length of the distal sensor signal wire(s) 28a. Conversely, the protective tube 30 may span a shorter length than the distal sensor signal wire(s) 28a, such as, for example, without limitation, from a position proximal of the proximal neck portion 16a of the occlusion balloon 16 to a position distal of the distal neck portion 16b of the occlusion balloon 16. In either configuration, the protective tube 30 may be affixed at the proximal and distal balloon neck portion 16a, 16b anchoring areas, e.g., via bonding (e.g., adhesive or thermal), welding, a combination thereof or the like, with the distal sensor signal wire(s) 28a extending therein in an untaught or floating manner. Additionally, or alternatively, the distal sensor signal wire(s) 28a, or the protective tube 30, may be jacketed to the stiffener member 24, 24' via a jacket tube 31. Similarly to the protective tube 30, the jacket tube 31 may extend at least along the axial extent of the occlusion balloon 16 or the entire length of the distal sensor signal wire(s) 28a.

In another configuration, as shown in Fig. 3, the stiffener member 24, 24' may alternatively be replaced with a hypotube 23. A primary difference between the hypotube 23 and the stiffener member 24, 24' is the presence of an internal lumen 23a axially extending through the hypotube 23. For the sake of brevity, other features of the hypotube 23 remain as described with respect to the stiffener member 24, 24'. For example, the hypotube 23 may take the same tapered external profile as the stiffener member 24 or may be of uniform external profile as the stiffener member 24' and overlap with the solid distal wire 25, as previously described. The hypotube 23 may also be anchored at a proximal end thereof within the display hub 22, as previously described. Where a hypotube 23 is employed, the distal sensor signal wire(s) 28a may route through the hypotube 23 to the display hub 22.

Turning to the occlusion balloon 16, in one configuration, the occlusion balloon 16 may be constructed of a semi-compliant or substantially non-compliant material. In one configuration, the entirety of the balloon 16 may be constructed of the same material, e.g., a polymeric semi-compliant or substantially non-compliant material. In an alternative configuration, the occlusion balloon 16 may be constructed of multiple polymeric materials. In the context of the present disclosure, a non-compliant or substantially non-compliant balloon generally has growth of approximately two to seven percent (2-7%) within the working range (balloon pressure) when inflated, a semi-compliant balloon has growth of approximately seven to twenty percent (7-20%) within the working range (balloon pressure) when inflated and a compliant balloon has growth of approximately greater than twenty percent (20%+) within the working range (balloon pressure) when inflated. Compliant balloons, in contrast, may have growth of approximately one hundred to three hundred percent (100-300%) within the working range (balloon pressure) when inflated.

As should be understood by those of ordinary skill in the art, ascertaining blood flow through the target vessel at the occlusion site is critical for conducting vascular occlusion related procedures. Directly measuring vascular blood flow in human patients via surgically placed perivascular flow probes is challenging, particularly in emergency or trauma environments where guidance imaging is typically not available. A direct relationship exists, however, between blood flow and below balloon blood pressure, i.e., blood pressure downstream of the occlusion balloon 16 measured by the proximal sensor 26. Accordingly, below balloon blood pressure may be relied upon as an indicator of flow, particularly in emergent or trauma situations. It is imperative, therefore, to ensure that such readings are accurate and reliable, eliminating, or at least minimizing, artifacts. As should be understood, the description of blood pressure herein encompasses systolic, diastolic or mean arterial blood pressure.

The inventors have discovered that when a balloon constructed of a thin, compliant material is positioned within a target vessel, e.g., the aorta, and inflated to fully occlude the vessel, the pulsatile blood pressure generated by the heart upstream of the balloon may transfer force/energy on the compliant balloon causing the compliant balloon to pulsate due to the elastic and stretchy material properties thereof. Accordingly, despite being fully occlusive, such balloon pulsations may impart a pulsatile pressure wave on the downstream blood, effectively transferring the upstream blood pressure pulsation from the upstream side thereof to the downstream side thereof. This results in a below balloon artificially-pulsatile blood pressure despite the absence of actual below balloon flow. Thus, stretchy compliant balloon material may transfer upstream pulsatile blood pressure onto the downstream side of the balloon without the actual presence of blood flow past the balloon. Accordingly, an operator/practitioner (hereinafter "practitioner"), whether experienced or novice, relying upon the downstream blood pressure reading as an indicator of blood flow may observe a reading of downstream blood pressure, falsely indicating that vessel is in a state of partial occlusion, i.e., indicating that the compliant occlusion balloon is partially occluding the vessel while permitting some blood flow past the balloon, when the vessel is actually in a state of full occlusion (the occlusion balloon is fully occluding the vessel). Such a situation may be very dangerous for a patient as the practitioner would otherwise believe that the vessel is only in a state of partial occlusion, allowing a greater duration of safe occlusion time, whereas the vessel is actually in a state of full occlusion, with a reduced duration of safe occlusion time (e.g., half or less than half of the time as partial occlusion). Advantageously, the inventors have found that the relatively inelastic material properties of a semi-compliant or substantially non-compliant balloon material make the balloon less susceptible to the aforementioned pulsatile artifact exhibited with compliant balloon material, at least in part because the more inelastic material of the balloon is less likely to absorb and release energy in response to upstream pulsatile blood pressure. Accordingly, the below balloon blood pressure reading is a more accurate indicator of blood flow downstream of the occlusion balloon.

The semi-compliant or substantially noncompliant occlusion balloon 16 may also be "oversized", i.e., define a blown diameter D that is larger than a diameter of the target, i.e., destination, blood vessel (not shown) into which the occlusion balloon 16 is inserted and inflated for occlusion. In one configuration, the occlusion balloon 16 may define a blown diameter D between approximately nine and approximately thirty-five millimeters (~9-35mm), which is configured to be at least approximately ten percent (10%) larger than the diameter of the target vessel, e.g., the aorta, in a normotensive state. As should be understood by those of ordinary skill in the art, aortic diameter ranges from between approximately 8 mm and approximately 26 mm, depending on the location along the aorta. The occlusion balloon 16 is, therefore, only partially inflated when its outer surface comes into full diametric contact/apposition with the inside wall of the target vessel and folds (not shown) remain at the peripheral surface of the occlusion balloon 16. The open/un-collapsed folds, in combination with the opposing inner wall of the vessel or with radially overlying portions of the outer surface of the occlusion balloon 16, result in flow channels (not shown) extending along the length of the occlusion balloon 16 that allow partial perfusion or blood flow past the occlusion balloon 16 under the blood pressure within the vessel, such as, for example, as described in the '895 publication.

Turning to the display hub 22 more specifically, which is integrated into the catheter system 10, a primary purpose of the display hub 22 is to display information and/or commands that facilitate a practitioner's successful operation of the catheter system 10, particularly in stressful emergency and trauma situations, without the benefit of imaging guidance, when practitioners may be more prone to overlook details.

As one example, the display hub 22 may be configured to notify whether the appropriate target vessel has been catheterized, e.g., whether or not an artery such as the aorta has been catheterized. In one configuration, for example, the display hub 22 may be configured to indicate non-arterial placement, e.g., in a vein or extravascular space, if the blood pressure detected (via sensor(s) 26, 28) is less than a static threshold, e.g., less than approximately 30 mmHg prior to balloon inflation. In such instance, a command field 34a (or an alternative notification field 34b) may display an alert, such as "Artery Not Detected". Alternatively, if the blood pressure detected is greater than or equal to the static threshold prior to balloon inflation, the command field 34a (or the alternative notification field 34b) may display a confirmation notification, such as "Artery Detected". The alerts/notifications may also be color-coded, e.g., red font for errors such as "Artery Not Detected" and green font conforming successfully completed steps such as "Artery Detected". Additionally, or alternatively, errors may be displayed in a flashing manner whereas successfully completed steps may be displayed as solid text. Additionally, or alternatively, the display hub 22 may project an audible tone or beep or a tactile feedback to alert the practitioner of the error.

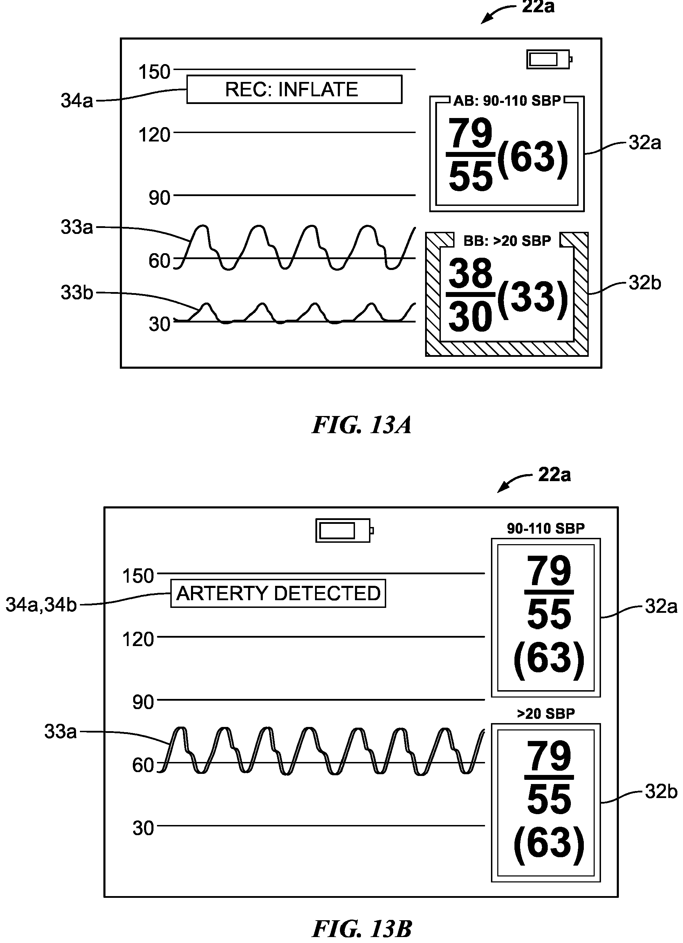

As another example, acceptable target ranges for above balloon (distal/upstream of the balloon when inserted in a retrograde manner) and below balloon (proximal/downstream of the balloon when inserted in a retrograde manner) blood pressures which enable safe, prolonged partial occlusion of an associated target vessel have been previously studied and published. Although live, detected blood pressure (via the proximal and/or distal pressure sensors 26, 28) output on the display screen 22a is beneficial to a practitioner, the practitioner must still remember, or otherwise ascertain, the desired target range(s). In one configuration, therefore, as shown in Figs. 13A-14, the preset target ranges for above balloon and below balloon blood pressures corresponding to a state of partial vessel occlusion may be output on the display screen 22a. As shown, for example, the target range for above balloon pressure may be between approximately 90 mmHg and approximately 110 mmHg systolic blood pressure or between approximately 55 mmHg and approximately 65 mmHg mean arterial pressure. In one example, the target range for below balloon pressure may be a pressure greater than approximately 20 mmHg systolic blood pressure or greater than approximately 20 mmHg mean arterial pressure. As should be understood, without limitation, diastolic blood pressure may additionally or alternatively be relied upon. Additionally, or alternatively, the display hub 22 may be configured to enable the practitioner to adjust the target range(s).

The display hub 22 may also be configured to clearly indicate whether the necessary physiologic response of a patient has been achieved after inflation of the occlusion balloon 16. As one example, when a practitioner inflates the occlusion balloon 16 with the intent to partially occlude the target vessel, e.g., the aorta, the display hub 22 may be configured to display at least one visual cue, e.g., in the form of a color-coded border 32a, 32b around the physiological parameter reading output, which indicates whether the enclosed parameter is within the target range. As one non-limiting example, a red border displayed around a numerical blood pressure reading may indicate that the pressure remains out of the target range for partial occlusion, whereas a green border displayed around the numerical blood pressure reading may indicate that the pressure is within the desired target range for partial occlusion. A yellow border may indicate that the physiological parameter is approaching an outer limit of the target range. Alternatively, rather than employing a color-coded border, a shaded color-coded shape (not shown) surrounding the associated numerical value may be employed to indicate whether the respective value is within the desired target range. Additionally or alternatively, color-coded font color of the associated numerical value may be employed to indicate whether the respective value is within the desired target range. Additionally or alternatively, text size changes of the associated numerical value may be employed to indicate whether the respective value is within the desired target range. Advantageously, the aforementioned visual cues are easily observable by a practitioner from afar, notifying the practitioner whether the regions of the body perfused upstream of the occlusion balloon as well as whether the regions of the body perfused downstream of the occlusion balloon are being adequately perfused for prolonged partial vessel occlusion.

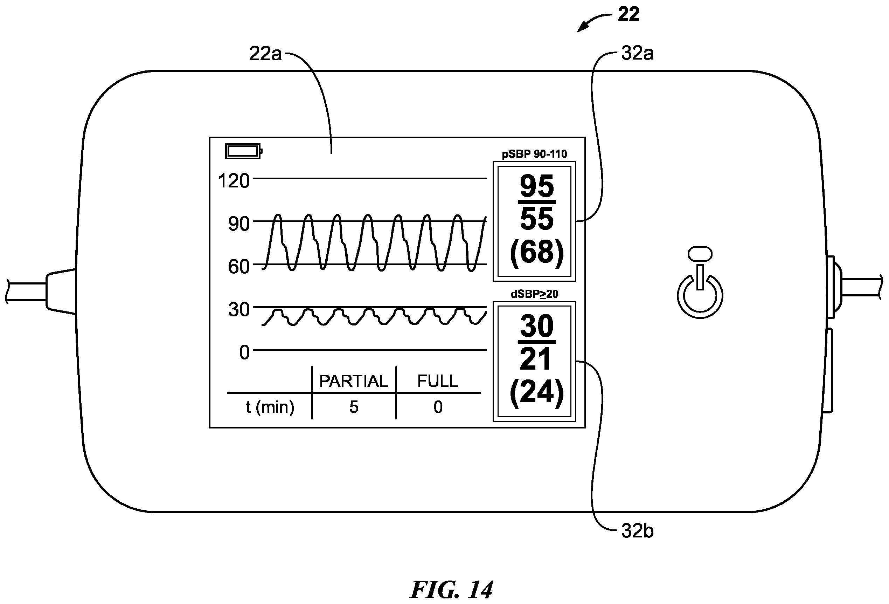

Additionally, or alternatively, other displays on the display screen 22a may be employed to further facilitate a successful procedure for the practitioner. As one example, the detected physiologic parameter may be displayed in the form of at least one color-coded waveform 33a, 33b which indicates whether the associated parameter is within the target range. As one non-limiting example, a red blood pressure waveform may indicate that the associated pressure (above and/or below balloon) remains out of the target range, whereas a green waveform may indicate that the associated pressure (above and/or below balloon) is within the desired target range. As should be understood by those of ordinary skill in the art, the peak of a pressure wave corresponds to the associated systolic blood pressure and the trough of a wave corresponds to the associated diastolic blood pressure. As shown in Figs. 13A and 14, both the above balloon physiological parameter (e.g., pressure) waveform and below balloon physiological parameter (e.g., pressure) waveform may be displayed on the same graph. Advantageously, in a state of no occlusion, the waveforms should nearly overlap, whereas in a state of partial vessel occlusion, the waveforms diverge from one another as the occlusion balloon 16 blocks blood flow. The waveforms may be displayed in different colors from one another. Additionally, or alternatively, the waveforms may match the color of the corresponding numerically displayed values. The display screen 22a may further be configured with additional or alternative features to provide prominent status indicating cues to a practitioner, including, but not limited to, a change in the font style (e.g., bold and/or italicized) of a numerical parameter display, the presence of a check mark or other text output, popup or banner upon achieving the desired target or target range, an audible alert, a tactile alert (e.g., vibration), flashing lights on the display hub 22a, combination thereof or the like.

In addition to prominent notifications indicating whether the requisite physiologic response of a patient has been achieved, the display hub 22 may be further configured to provide instructions, e.g., visual commands, audible commands or other sound, according to the sensed physiological parameter(s) (via sensor(s) 26, 28) to assist the practitioner to more efficiently achieve the physiologic parameter(s) target(s). As one non-limiting example, as shown in Fig. 13A, the display screen 22a may include a command field 34a which may display a command for the practitioner according to the respectively detected physiologic parameters, e.g., above and/or below balloon blood pressure, relative to the pre-determined targets or target ranges. For example, if the above balloon pressure is less than a respective threshold value, the command field 34a may display the command "Inflate" or the like. As should be understood, if the above balloon pressure reading remains less than the target range, then the occlusion balloon 16 may be allowing toomuch blood to flow by, and, therefore, needs further inflation. Conversely, if the above balloon pressure is greater than a respective threshold value, or if the below balloon pressure is less than a respective threshold value,the command field 34a may display the command "Deflate", "Reduce Inflation" or the like. As should be understood, if the practitioner intends to employ the occlusion balloon 16 to partially occlude the target vessel, i.e., reduce blood flow past the balloon while permitting some blood flow by, then a below balloon pressure reading that is less than the target range indicates that the occlusion balloon 16 may be inflated more than desired, thereby occluding the vessel more than desired. Likewise, if the above balloon pressure is greater than the target, then the occlusion balloon 16 may also be inflated more than desired.

If the above balloon pressure and the below balloon pressure are both within the associated target ranges, the command field 34a may display the command "Hold" or the like, indicating to the practitioner that an appropriate level of balloon inflation has been reached. If, however, the above balloon pressure and the below balloon pressure are both below the target range, the command field 34a may display the command "Give Blood". In such a situation, the patient may require a blood infusion or transfusion. As should be understood by those of ordinary skill in the art, other commands may be employed, such as, without limitation "Above Balloon in Range", "Above Balloon out of Range", "Below Balloon in Range", "Below Balloon out of Range", a combination thereof, or the like.

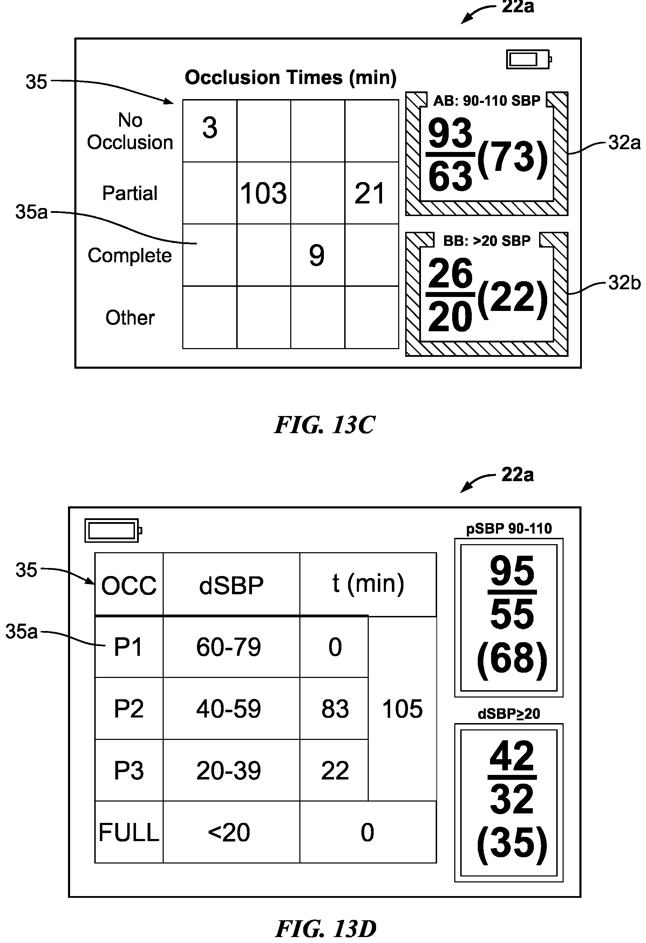

The display hub 22 may include other features intended to further inform and assist a practitioner. As one example, the display hub 22 may employ an internal, segmented timer (Fig. 13C). Relying upon the data received from the segmented timer and the proximal and distal sensors 26, 28, the display hub 22 is configured to record occlusions times and divide, categorize and sequentially/chronologically track duration of time in each occlusion state (including a state of no occlusion). This data may assist a practitioner when switching between complete and partial vessel occlusion states during the same procedure, as well as a state of no occlusion, according to the patient's particular condition or response to the vascular occlusion procedure. More specifically, not every emergency or trauma circumstance can afford employing only partial occlusion. Some patient conditions may require a combination of periods of complete occlusion, partial occlusion and/or no occlusion. Sequential/chronological tracking of physiological data is helpful for ascertaining the impact of the occlusion procedure on organ perfusion. By intermittently relieving complete vessel occlusion with periods of partial or no occlusion, for example, the overall procedure can be safely prolonged, while still protecting extremities and organs perfused downstream of the occlusion site. In such cases, tracking and displaying the duration of time a patient spends in each occlusion state, or lack thereof, in sequential order is beneficial to the practitioner.

As one, non-limiting, example, a practitioner may conduct a procedure that involves thirty minutes of complete occlusion, followed by ten minutes of no occlusion, followed by thirty minutes of complete occlusion, followed by ten minutes of no occlusion, followed by thirty minutes of complete occlusion. A "total elapsed timer" capable of only tracking total time is limited in tracking of such data, e.g., presenting the data as ninety minutes of complete occlusion and twenty minutes of no occlusion. A basic indication of ninety minutes of full occlusion may be inadequate, as such duration may be considered unsafe without accounting for the intermittent nature of the complete occlusion applied. Advantageously, in addition to tracking total elapsed time in a particular occlusion state, the segmented timer is further capable of breaking down total duration into categorized, sequential segments or intervals (see Fig. 13C). The segmented timer, therefore, in combination with the physiological parameter readings (e.g., pressure readings via sensor(s) 26, 28), enables the display hub 22 to display categorized durations of each occlusion category sequentially. For the aforementioned example above, the display hub 22 may accurately present this data as thirty minutes of complete occlusion, followed by ten minutes of no occlusion, followed by thirty minutes of complete occlusion, followed by ten minutes of no occlusion, followed by thirty minutes of complete occlusion.

More specifically, the display hub 22 may be configured to continuously or periodically classify pressure readings (above and/or below balloon blood pressure measured by the sensors 28, 26, respectively) with the respective appropriate occlusion categories (as described in further detail below), i.e., no vessel occlusion, partial vessel occlusion, complete vessel occlusion or other. As one example, for each predetermined duration of time, e.g., one-minute, the category with the highest elapsed time during that duration will be indexed. For example, a one-minute interval containing ten seconds of no occlusion, thirty seconds of partial occlusion, ten seconds of complete occlusion and ten seconds of "other", may be indexed as one minute of partial occlusion. When a previous minute's occlusion category shifts to a new category, a new segment is added to index the category change and ultimately provide a sequential log of how long the patient spent in each occlusion state. Alternatively, as another example, the display hub 22 may be configured to continuously classify pressure readings with respective appropriate occlusion categories and maintain an occlusion category so long as the pressure readings continue qualifying for the respective category. If/once the pressure readings change to a different occlusion category, a new segment is added to index the occlusion category change and provide a chronological log of how long the patient experienced each category. Additionally, or alternatively, the display hub 22 may also be configured to count and output the total time the patient has been exposed to each state of occlusion, as shown in Fig. 14. Additionally, or alternatively, the display hub 22 may be configured to count down and display the remaining safe exposure time for each state of vessel occlusion.

In one non-limiting configuration, the display hub 22 may be configured to classify pressure readings as "no vessel occlusion" when the absolute value difference between above and below balloon blood pressures is less than a predetermined value. As one example, if the difference between above and below balloon blood pressures is less than approximately 10 mmHg, the display hub 22 may be configured to classify such state as no occlusion.

In one non-limiting configuration, the display hub 22 may be configured to classify pressure readings as "partial vessel occlusion" when the above balloon blood pressure is between a designated range in combination with the below balloon blood pressure being at least a certain threshold pressure. As one example, if the above balloon systolic blood pressure is between approximately 90 mmHg and approximately 110 mmHg in combination with the below balloon systolic blood pressure being at least approximately 20 mmHg, such state may be categorized as partial occlusion. Alternatively, in another non-limiting configuration, the display hub 22 may be configured to classify pressure readings as "partial vessel occlusion" when above balloon blood pressure is at least one associated threshold pressure, in combination with the below balloon blood pressure being at least another associated threshold pressure. As one example, if the above balloon systolic blood pressure is at least approximately 90 mmHg, in combination with the below balloon systolic blood pressure being at least approximately 20 mmHg, such state may be categorized as partial occlusion.

In yet another non-limiting configuration, the display hub 22 may be configured to classify pressure readings as "partial vessel occlusion" when at least one of: (i) there is a difference between the above and below balloon pressures; (ii) the below balloon blood pressure is pulsatile; and (iii) the below balloon blood pressure indicates flow. For example, the display hub 22 may be configured to classify pressure readings as "partial vessel occlusion" when at least one of: (i) the difference between the above and below balloon systolic pressures is at least approximately 10 mmHg; (ii) the below balloon systolic pressure is at least approximately 5 mmHg greater than the below balloon diastolic pressure; and (iii) the below balloon systolic blood pressure is greater than approximately 20 mmHg.

In one non-limiting configuration, the display hub 22 may be configured to classify pressure readings as "complete vessel occlusion" when the below balloon blood pressure is less than a threshold pressure. As one example, if the below balloon systolic blood pressure is below approximately 20 mmHg, such state may be categorized as complete occlusion. In one non-limiting configuration, the display hub 22 may be configured to classify pressure readings as "other" when any combination of pressures that does not meet the criteria for no vessel occlusion, partial vessel occlusion or complete vessel occlusion.

In another non-limiting configuration, the display hub 22 may be configured to classify pressure readings as "complete vessel occlusion" when at least one of: (i) the below balloon blood pressure is non-pulsatile, or (ii) the below balloon blood pressure is low enough to indicate no flow. For example, the display hub 22 may be configured to classify pressure readings as "complete vessel occlusion" when at least one of: (i) the difference between the below balloon systolic pressure and the below balloon diastolic pressure is less than approximately 5 mmHg; or (ii) the below balloon systolic pressure is approximately 20 mmHg or less.

As shown in Fig. 13C, the timer display on the display screen 22a may include a grid 35, e.g., a timetable, logging the number of minutes spent in each category, in chronological order. In the illustrated embodiment of Fig. 13C, each occlusion category occupies a row of cells 35a on the grid 35, whereas the columns from left to right indicate chronological order. A numerical time value is displayed in each appropriate cell indicating the number of minutes spent in the associated occlusion category. As should be understood, however, this data may additionally or alternatively be presented in the form of a chart or a graph. Optionally, the display hub 22 may be configured with respective threshold, continuous occlusion durations considered to be safe for one or both of partial vessel occlusion and complete vessel occlusion. For example, a continuous one-hundred-twenty minutes may be considered the maximum safe continuous duration for partial occlusion. As another example, a continuous duration of thirty minutes may be considered the maximum safe continuous duration for complete occlusion. Optionally, the safe upper limit of the respective continuous durations may be displayed on the display screen 22a to remind a practitioner. In one configuration, the numeric values and/or the numbered cells, at least within the partial and/or complete occlusion rows, may be color-coded to indicate whether each recorded duration fell within the safe limit. For example, a numeric value or cell could be color-coded green for durations considered safe and color-coded red for durations considered unsafe. An optional notification/alarm could sound when the safe limit is approaching or exceeded.

Optionally, as shown in Fig. 13D, in addition to displaying the total time spent in a state of partial occlusion, the display hub 22 can also be configured to track and display the duration of partial vessel occlusion, further categorized into preset grades. Breaking down partial occlusion into grades may help identify the optimal distal blood pressure range. In addition to tracking total partial vessel occlusion duration, the segmented timer may also track the duration spent in each preset grade of partial occlusion. In the illustrated embodiment, P1 may define the grade of partial occlusion with the highest below balloon flow, i.e., the highest below balloon blood pressure, whereas P3 may define the grade of partial occlusion with the least below balloon flow, i.e., the lowest below balloon blood pressure.

In one non-limiting configuration, the display hub 22 may be configured to classify partial vessel occlusion as grade 1 when the aforementioned partial vessel occlusion parameter(s) is/are met and the below balloon blood pressure indicates high flow range, e.g., a systolic blood pressure between approximately 50 mmHg and approximately 75 mmHg. In one non-limiting configuration, the display hub 22 may be configured to classify partial vessel occlusion as grade 2 when the aforementioned partial vessel occlusion parameter(s) is/are met and the below balloon blood pressure indicates medium flow range, e.g., a systolic blood pressure between approximately 35 mmHg and approximately 49 mmHg. In one non-limiting configuration, the display hub 22 may be configured to classify partial vessel occlusion as grade 3 when the aforementioned partial vessel occlusion parameter(s) is/are met and the below balloon blood pressure indicates low flow range, e.g., a systolic blood pressure between approximately 21 mmHg and approximately 34 mmHg. The current or live grade of partial occlusion may be displayed in a unique manner, .e.g., without limitation, in a different color, have a surrounding colored box, in a highlighted manner, or the like.

Advantageously, the aforementioned detailed data provides the practitioner with a better understanding of the level of perfusion downstream organs and extremities have received during the vascular occlusion procedure. The display hub 22 may include at least one physical button (not shown) and/or the display screen 22a, taking the form of a touchscreen, may include at least one virtual button (not shown), for selective toggling between screens, e.g., between the graphical view of the waveforms and the occlusion timetable.

It will be appreciated by those skilled in the art that changes could be made to the embodiment(s) described above without departing from the broad inventive concept thereof. It is understood, therefore, that this disclosure is not limited to the particular embodiments disclosed, but it is intended to cover modifications within the spirit and scope of the present disclosure as defined by the present description, as set forth in the appended claims.

## Claims

1. A vascular occlusion catheter system for at least partial occlusion of a target vessel, the vascular occlusion catheter system comprising:
a proximal catheter tubular member;
a distal catheter tubular member;
an intervening occlusion balloon connected to the proximal catheter tubular member and the distal catheter tubular member;
one of a stiffener member or a hypotube extending within the proximal catheter tubular member, the occlusion balloon and into the distal catheter tubular member, the stiffener member or the hypotube being permanently anchored within the vascular occlusion catheter system;
a display hub fixedly positioned upon the proximal catheter tubular member, the display hub having a display screen;
a proximal pressure sensor positioned within the proximal catheter tubular member and in fluid communication with an environment external to the vascular occlusion catheter system proximal of the occlusion balloon, the proximal pressure sensor being electrically connected with the display hub; and
a distal pressure sensor positioned within the distal catheter tubular member and in fluid communication with an environment external to the vascular occlusion catheter system distal of the occlusion balloon, the distal pressure sensor being electrically connected with the display hub,
wherein the display hub is: (i) preset with an above balloon target blood pressure range and a below balloon target blood pressure range each associated with safe partial occlusion of the target vessel, (ii) configured to output a live proximal pressure sensor reading and a live distal pressure sensor reading on the display screen, (iii) configured to display a visual cue on the display screen indicating whether the live distal pressure sensor reading is within the above balloon target blood pressure range, and (iv) configured to display a visual cue on the display screen indicating whether the live proximal pressure sensor reading is within the below balloon target blood pressure range.

2. The vascular occlusion catheter system of claim 1,
wherein the live distal pressure sensor reading output comprises a first numerical output and the live proximal pressure sensor reading output comprises a second numerical output,
wherein the visual cue indicating whether the live distal pressure sensor reading is within the above balloon target blood pressure range comprises at least one of (i) a colored border around the first numerical output, (ii) a shaded color-coded shape surrounding the first numerical output, or (iii) a color-coded font color of the first numerical output, and
wherein the visual cue indicating whether the live proximal pressure sensor reading is within the below balloon target blood pressure range comprises at least one of (i) a colored border around the second numerical output, (ii) a shaded color-coded shape surrounding the second numerical output, or (iii) a color-coded font color of the second numerical output.

3. The vascular occlusion catheter system of claim 1 or claim 2, wherein the live distal pressure sensor reading output comprises a first waveform and the live proximal pressure sensor reading output comprises a second waveform, the visual cue indicating whether the live distal pressure sensor reading is within the above balloon target blood pressure range comprises a color-coded output of the first waveform and the visual cue indicating whether the live proximal pressure sensor reading is within the below balloon target blood pressure range comprises a color-coded output of the second waveform.

4. The vascular occlusion catheter system of claim 1 or claim 2, wherein the live distal pressure sensor reading output comprises a first waveform and the live proximal pressure sensor reading output comprises a second waveform, and the first waveform and the second waveform are output on a single graph.

5. The vascular occlusion catheter system of any one of the previous claims, wherein at least one of the above balloon target blood pressure range and the below balloon target blood pressure range is output on the display screen.

6. The vascular occlusion catheter system of any one of the previous claims, wherein the display hub includes a segmented timer, the display hub being configured to record occlusions times and divide, categorize and chronologically track duration of time in a particular occlusion state, or a particular grade of an occlusion state, according to data received from the segmented timer, the proximal pressure sensor and the distal pressure sensor.

7. The vascular occlusion catheter system of claim 6, wherein the display hub is configured to classify proximal and distal pressure sensor readings as a no vessel occlusion, occlusion state when the absolute value difference between distal and proximal blood pressure sensor readings is less than approximately 10 mmHg.

8. The vascular occlusion catheter system of claim 6 or claim 7, wherein the display hub is configured to classify proximal and distal pressure sensor readings as a partial vessel occlusion, occlusion state when (i) the distal pressure sensor reading is between approximately 90 mmHg and approximately 110 mmHg in combination with the proximal pressure sensor reading being at least approximately 20 mmHg, or (ii) the distal pressure sensor reading is at least approximately 90 mmHg in combination with the proximal pressure sensor reading being at least approximately 20 mmHg.

9. The vascular occlusion catheter system of claim 6 or claim 7, wherein the display hub is configured to classify proximal and distal pressure sensor readings as a partial vessel occlusion, occlusion state when at least one of: (i) the absolute value difference between distal and proximal blood pressure sensor readings is at least approximately 10 mmHg; (ii) a systolic proximal pressure sensor reading is at least 5 mmHg greater than a diastolic proximal pressure sensor reading; and (iii) the proximal pressure sensor reading being greater than approximately 20 mmHg.

10. The vascular occlusion catheter system of claim 8 or claim 9, wherein the display hub is configured to:
classify proximal and distal pressure sensor readings as a first grade of partial vessel occlusion when the proximal pressure sensor reading is between approximately 50 mmHg and approximately 75 mmHg;
classify proximal and distal pressure sensor readings as a second grade of partial vessel occlusion when the proximal pressure sensor reading is between approximately 35 mmHg and approximately 49 mmHg; and
classify proximal and distal pressure sensor readings as a third grade of partial vessel occlusion when the proximal pressure sensor reading is between approximately 21 mmHg and approximately 34 mmHg.

11. The vascular occlusion catheter system of any one of claims 6 to 10, wherein the display hub is configured to classify proximal and distal pressure sensor readings as a complete vessel occlusion, occlusion state when at least one of: (i) a systolic proximal pressure sensor reading is less than approximately 5 mmHg greater than a diastolic proximal pressure sensor reading; and (ii) the proximal pressure sensor reading is approximately 20 mmHg or less.

12. The vascular occlusion catheter system of any one of claims 6 to 11, wherein the display hub is configured to output a timer display as a grid logging a duration of time spent in each occlusion state or grade of the occlusion state.

13. The vascular occlusion catheter system of any one of the previous claims, wherein the display hub is configured to display an alert notifying whether an artery is detected according to a comparison of at least one of the proximal pressure sensor reading and the distal pressure sensor reading to a preset threshold pressure associated with the target artery, prior to inflation of the occlusion balloon.

14. The vascular occlusion catheter system of claim 13, wherein the display screen includes a command field and wherein the alert is a text alert displayed within the command field.

15. The vascular occlusion catheter system of any one of the previous claims, comprising one of:
(i) a stiffener member defining at least one tapered section in a direction from a proximal side toward a distal side thereof;
(ii) a stiffener member having a substantially uniform diameter throughout an axial extent thereof, and further comprising a solid distal wire extending within the distal catheter tubular member, a section of the solid distal wire overlapping with a section of the stiffener member, whereby the solid distal wire has a central axis that is radially offset from a central axis of the stiffener member; or
(iii) a hypotube, wherein the distal pressure sensor is electrically connected with the display via an internal lumen of the hypotube.
